# EUROPEAN PATENT APPLICATION

(11) **EP 3 549 638 A2**
(43) Date of publication of application: **09.10.2019**
(21) Application number: 19167484.5
(22) Date of filing: 05.04.2019
(51) Int. Cl.: A61P 43/00, A61K 31/191

(54) **PHARMACEUTICAL COMPOSITION FOR USE IN TREATING AN AGE-ASSOCIATED CONDITION**

(30) Priority: 06.04.2018 EP 18166074
(71) Applicant: Tomorrowlabs GmbH, 1010 Wien (AT)
(72) Inventor: Duscher, Dominik, 81675 München (DE); Thor, Dominik, 1010 Wien (AT)
(74) Representative: Zwicker, Jörk

(57) **Abstract**

The present invention relates to the field of pharmacology and in particular to a pharmaceutical composition for use in treating an age-associated condition. More specifically, the present invention relates to a pharmaceutical composition comprising a gluconic acid salt for use in treating an age-associated condition caused by cellular stress. The invention also relates to the use of a pharmaceutical composition comprising a gluconic acid for the cosmetic treatment of the ageing skin.

## Description

### FIELD OF THE INVENTION

The present invention relates to the field of pharmacology and in particular to a pharmaceutical composition for use in treating an age-associated condition. More specifically, the present invention relates to a pharmaceutical composition comprising a gluconic acid salt for use in treating an age-associated condition caused by cellular stress. The invention also relates to the use of a pharmaceutical composition comprising a gluconic acid for the cosmetic treatment of the ageing skin.

### BACKGROUND OF THE INVENTION

Aging is associated with mitochondrial dysfunction, leading to reduced respiratory metabolism and increased generation of reactive oxygen species (ROS) in cells. Persistent DNA damage may arise from both increased oxidative damage and reduced efficiency of energy-intensive DNA repair, predisposing to apoptosis, senescence, and inflammation. Aging is also associated with increased protein misfolding and aggregation in the cytoplasm, nucleus, and endoplasmic reticulum. The various sites of age-related cellular damage and the physiological decline that ensues contribute to the pathogenesis of age-related diseases, including metabolic syndrome, inflammatory disorders, cancer, and neurodegenerative diseases (Haigis C. et al., Mol Cell. 2010 Oct 22; 40(2): 333-344).

Although the causes of many age-related diseases remain unknown, cellular stress is thought to be the main culprit. Countless physical and chemical factors are known to induce distinct cellular stress that can manifest in a cell population in a variety of ways including DNA damage, oxidative stress, energy transforming and redox state. Oxidative stress, for example, is often defined as an imbalance of pro- and antioxidants which can lead to damage of cellular lipids, proteins and DNA. This stress is known to affect various cellular functions including gene transcription, cell proliferation and differentiation and can lead to various disorders and diseases such as cardiovascular and neurodegenerative diseases including diabetes, hypertension and age-related cancers.

Age-related diseases create a growing economic burden on health care systems as the population grows older. Because of its involvement in many age-related disease processes, cellular stress is a promising target for treating aging and age-related diseases as well as disease therapy.

Accordingly, it is an object of the present invention to provide a pharmaceutical composition which effectively inhibits the pathogenesis of age-related diseases. More specifically, it is an object of the invention to provide a pharmaceutical composition which effectively inhibits the pathogenesis of age-related diseases by ameliorating cellular stress.

It is also an object of the present invention to provide a pharmaceutical composition which ameliorates cellular stress in cells of the skin. Such a pharmaceutical composition could not only be used for inhibiting the pathogenesis of age-related skin conditions and diseases such as dry skin, itching, ulcers, etc., but would also be a powerful tool for preventing signs of skin aging such as wrinkles sagging and relaxing of the cutaneous tissue.

These objects are surprisingly met by the present invention.

### SUMMARY OF THE INVENTION

**In a first aspect**, the present invention provides a gluconic acid salt for use in treating an age-associated condition caused by cellular stress in a subject.
In an embodiment of the pharmaceutical composition for use according to the first aspect, cellular stress comprises release of lactate dehydrogenase (LDH) from a cell. Preferably, said cell is a skin cell, more preferably a keratinocyte, fibroblast and/or endothelial cell.

In a further embodiment of the pharmaceutical composition for use according to the first aspect, said use comprises topically administering the pharmaceutical composition to the subject.

In a further embodiment of the pharmaceutical composition for use according to the first aspect, said use comprises injecting the pharmaceutical composition into the epidermis, dermis or subcutis of the subject, preferably into the dermis, most preferably into the *stratum reticulare.*

In an embodiment of the pharmaceutical composition for use according to the first aspect, the gluconic acid salt is sodium gluconate.

In a further embodiment of the pharmaceutical composition for use according to the first aspect, the gluconic acid salt is comprised in the composition according to the first aspect at a concentration of from 0.01 to 10% by weight, from 0.05 to 9% by weight, from 0.1 to 6% by weight, from 0.5 to 4% by weight, or from 1 to 2% by weight of said composition, and preferably from 0.05 to 0.1% by weight or from 0.5 to 1% by weight of said composition.

In a further embodiment, the pharmaceutical composition for use according to the first aspect is selected from the group consisting of a cutaneous cream, a cutaneous ointment, a cutaneous gel, a cutaneous plaster, a cutaneous foam, a cutaneous spray, a cutaneous paste, a cutaneous powder, a cutaneous emulsion, a cutaneous solution, a cutaneous suspension, a solution for injection, a suspension for injection, an emulsion for injection, and a gel for injection.

In a further embodiment, the pharmaceutical composition for use according to the first aspect is a slow-release composition.

In a further embodiment, the pharmaceutical composition for use according to the first aspect further comprises a natural oil component selected from soy oil, argan oil, shea butter, almond oil and combinations thereof, and/or a further component selected from hyaluronic acid, collagen, nicotin amide, provitamin B5, moringa extract, vitamin E, a UV filter, and combinations thereof.

In a further embodiment, the pharmaceutical composition for use according to the first aspect further comprises vitamin C and/ or vitamin A, preferably all trans retinyl palmitate.

In a further embodiment, the pharmaceutical composition for use according to the first aspect further comprises a preserving agent, preferably selected from the group consisting of phenoxyethanol, benzoic acid, dehydroacetic acid, ethylhexylglycerin, sodium benzoate and combinations thereof.

In a further embodiment, the pharmaceutical composition for use according to the first aspect further comprises a skin penetration enhancing agent. Said skin penetration enhancing agent is preferably selected from the group consisting of a C12-C15 alkyl benzoate, pentylene glycol, polyethylene glycol, ethoxydiglycol, dimethyl sulfoxide, sodium lauryl sulfate, polysorbate polyethylene sorbitane monolaurate, lecithin and mixtures thereof.

**In a second aspect**, the present invention provides a pharmaceutical composition comprising sodium gluconate, a natural oil component selected from soy oil, argan oil, shea butter, almond oil and combinations thereof, and/or a further component selected from hyaluronic acid, collagen, nicotin amide, provitamin B5, moringa extract, vitamin E, a UV filter, and combinations thereof.

In an embodiment of the pharmaceutical composition according to the second aspect of the invention, sodium gluconate is comprised in said composition at a concentration of from 0.01 to 10% by weight, from 0.05 to 9% by weight, from 0.1 to 6% by weight, from 0.5 to 4% by weight, or from 1 to 2% by weight of said composition, and preferably from 0.05 to 0.1% by weight or from 0.5 to 1% by weight of said composition..

In a further embodiment, the pharmaceutical composition according to the second aspect is selected from the group consisting of a cutaneous cream, a cutaneous ointment, a cutaneous gel, a cutaneous plaster, a cutaneous foam, a cutaneous spray, a cutaneous paste, a cutaneous powder, a cutaneous emulsion, a cutaneous solution, a cutaneous suspension, a solution for injection, a suspension for injection, an emulsion for injection, and a gel for injection.

In a further embodiment, the pharmaceutical composition according to the second aspect is a slow-release composition.

In a further embodiment, the pharmaceutical composition according to the second aspect further comprises vitamin C and/ or vitamin A, preferably all trans retinyl palmitate.

In a further embodiment, the pharmaceutical composition according to the second aspect further comprises a preserving agent, preferably selected from the group consisting of phenoxyethanol, benzoic acid, dehydroacetic acid, ethylhexylglycerin, sodium benzoate and combinations thereof.

In a further embodiment, the pharmaceutical composition according to the second aspect further comprises a skin penetration enhancing agent, which is preferably selected from the group consisting of a C12-C15 alkyl benzoate, pentylene glycol, polyethylene glycol, ethoxydiglycol, dimethyl sulfoxide, sodium lauryl sulfate, polysorbate polyethylene sorbitane monolaurate, lecithin and mixtures thereof.

**In a third aspect**, the present invention relates to the use of a pharmaceutical composition according to the second aspect to cosmetically treat a healthy area of skin of an individual, preferably wherein said individual is an aging individual.

In an embodiment, the use according to the third aspect of the invention comprises topically administering the pharmaceutical composition to the subject.
In a further embodiment, said use comprises injecting the pharmaceutical composition into the epidermis, dermis or subcutis of the subject, preferably into the dermis, most preferably into the *stratum reticulare.*

### FIGURES

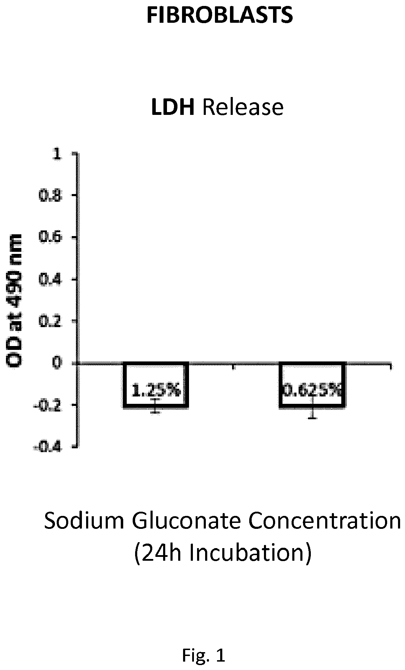

**Fig. 1****: Graphical representation of LDH release from human fibroblasts after treatment with sodium gluconate.** The graphs illustrate the results from the LDH release assay described in Example 1. Human fibroblasts were treated with sodium gluconate at concentrations of 0.625% and 1.25% by weight.

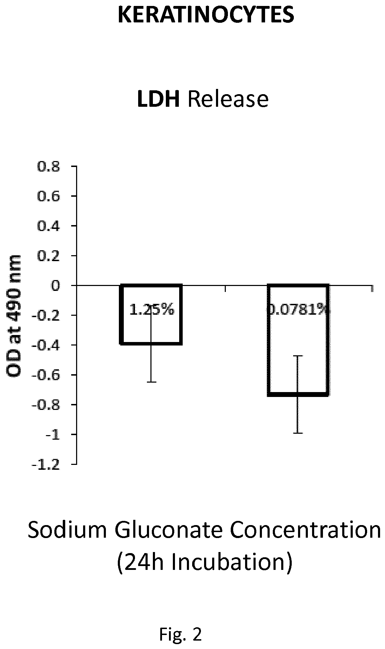

**Fig. 2****: Graphical representation of LDH release from human keratinocytes after treatment with sodium gluconate.** The graphs illustrate the results from the LDH release assay described in Example 2. Human keratinocytes were treated with sodium gluconate at concentrations of 0.0781% and 1.25% by weight.

### DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Several documents are cited throughout the text of this specification. Each of the documents cited herein (including all patents, patent applications, scientific publications, manufacturer's specifications, instructions etc.), whether supra or infra, is hereby incorporated by reference in its entirety. Nothing herein is to be construed as an admission that the invention is not entitled to antedate such disclosure by virtue of prior invention.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the word "comprise", and variations such as "comprises" and "comprising", are to be understood to imply the inclusion of a stated integer or step or group of integers or steps but not the exclusion of any other integer or step or group of integer or step. As used in this specification and the appended claims, the singular forms "a", "an", and "the" include plural referents, unless the content clearly dictates otherwise.

### DETAILED DESCRIPTION OF THE INVENTION

In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

In the work leading to the present invention it was surprisingly found that administering a gluconic acid salt to a subject effectively treats age-associated conditions in said subject. Specifically, it was surprisingly found by the present inventors that said gluconic acid salt effectively treats age-associated conditions that are caused by cellular stress.

Accordingly, the present invention provides in a first aspect a gluconic acid salt for use in treating an age-associated condition caused by cellular stress in a subject.

### Gluconic acid salts

Gluconic acid or (2R,3S,4R,5R)-2,3,4,5,6-pentahydroxyhexanoic acid (IUPAC) is an organic compound with the molecular formula C₆H₁₂O₇ and condensed structural formula HOCH₂(CHOH)₄COOH. The salts and esters of gluconic acid are known as "gluconates".

Gluconic acid is a multifunction carbonic acid. Due to their physiological and chemical characteristics, gluconic acid salts are used extensively in various pharmaceutical applications. For instance, calcium gluconate, in the form of a gel, is used to treat burns from hydrofluoric acid. Calcium Gluconate injections may be used for more severe cases to avoid necrosis of deep tissues. Potassium gluconate is administered in all cases of potassium deficiency and when there is danger of potassium depletion, which may be caused by diabetic acidosis, diarrhea, vomiting etc. Quinine Gluconate is a salt between gluconic acid and quinine, which is used for intramuscular injection in the treatment of malaria. Zinc Gluconate injections are used to neuter male dogs. Iron Gluconate injections have been proposed in the past to treat anemia.

As used herein, the term "gluconic acid salt" refers to any metal salt of gluconic acid known in the art. In the context of the present disclosure, the gluconic acid salt is preferably selected from the group consisting of sodium gluconate, potassium gluconate, calcium gluconate, magnesium gluconate, zinc gluconate, copper gluconate, ferrous gluconate, manganese gluconate and combinations thereof.

In a particularly preferred embodiment, the gluconic acid salt is sodium gluconate, which has the molecular formula NaC₆H₁₁O₇. Sodium gluconate chelates and forms stable complexes with various ions, preventing them from engaging in chemical reactions. Sodium gluconate is a safe and organic alternative to the synthetic chelating agents EDTA and etidronate to protect cosmetic products from discoloration and rancidity of cosmetic oils. It can also act as pH regulator as well as humectant, thanks to its molecular structure with numerous hydroxyl groups. Therefore, sodium gluconate is commonly used as a chelating agent, processing aid and stabilizing additive in medical, cosmetic and food applications.

### Treatment of age-associated conditions caused by cellular stress

Surprisingly, the present inventors discovered that sodium gluconate is highly effective in treating age-associated conditions caused by cellular stress in a subject and, thus, can also be used in a pharmaceutical composition as an active ingredient.

In the context of the present invention, the term "subject" is understood to include a mammal such as an animal and, more preferably, a human that is receiving or intended to receive treatment, as it is herein defined. While the term "subject" is often used herein to refer to a human, the invention is not so limited. Accordingly, the term "subject" refers to any animal, mammal or human having or at risk for a medical condition that can benefit from the treatment.

In a preferred embodiment, the subject is an aging subject, and, preferably, an aging human. In the present context, the term "aging human" is intended to refer to a human who is at least 30 years, preferably at least 40 years, more preferably at least 50 years, even more preferably at least 60 years, in particular at least 70 years, or even at least 80 years of age.

As used herein, the terms "treats", "treating", "treatment" and "to treat" include both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a diagnosed pathologic condition or disorder; and treatment of subjects at risk of contracting a disease or suspected to have contracted a disease, as well as subjects who are ill or have been diagnosed as suffering from a disease or medical condition. The term does not necessarily imply that a subject is treated until total recovery. The terms "treats", "treating", "treatment" and "to treat" also refer to the maintenance and/or promotion of health in an individual not suffering from a disease but who may be susceptible to the development of an unhealthy condition. The terms "treatment," "treating" and "to treat" are also intended to include the enhancement of one or more primary prophylactic or therapeutic measures.

In the context of the present invention, the term "cellular stress" is to be understood as any noxious factor (physical, chemical or biological), which triggers a series of cellular and systemic events, resulting in restoration of cellular and organismal homeostasis. Preferably, the term "cellular stress" as used herein refers to cellular stress caused by aging, exogenous hazards, such as UV radiation, endogenous toxic by-products of cellular metabolism, such as reactive oxygen species (ROS) and/or spontaneous chemical reactions, such as hydrolysis. Most preferably, "cellular stress" as used herein refers to cellular stress caused by aging.

Compromised cellular stress responses are linked to the onset of many age-associated conditions, including but not limited to cancer and age-related cardiovascular and neurodegenerative diseases, such as diabetes, hypertension, Alzheimer's, Parkinson's and Huntington's disease and amyotrophic lateral sclerosis.

The term "age-associated condition", as used herein, preferably refers to an age-related skin condition or disease, which is preferably selected from dry skin, itching, ulcers, fungal infections, benign and malignant tumors, pigmentation disorders such as melisma, and combinations thereof.

Accordingly, maintaining efficient mechanisms for counterbalancing cellular stress is emerging as a potential strategy towards ameliorating age-associated pathologies.

### Release of lactate dehydrogenase (LDH)

Such strategy for ameliorating age-associated pathologies by reducing cellular stress is provided by use of the pharmaceutical composition of the present invention. Specifically, the gluconic acid salt comprised in the inventive composition was found to effectively reduce cellular stress in a subject, wherein, preferably, said cellular stress comprises the release of lactate dehydrogenase (LDH) from a cell.

Lactate dehydrogenase (LDH) is a stable cytoplasmic enzyme present in all cells of the human or animal body. Enhanced glycolytic production of lactate is caused by hypoxia and oxidative stress and is likely a consequence of converging molecular and functional disturbances, which allow the cell to maintain ATP concentrations through the non-aerobic pathway. Therefore, enhanced release of lactate dehydrogenase (LDH) from a cell is a marker of impaired energy metabolism in any kind of cells. Moreover, since many molecular pathways converge on the glycolytic pathway, enhanced lactate production and release is considered as a global marker of sub-lethal injury and thus reported an ideal marker for investigating cell stress *in vivo* and *in vitro* (Limonciel et al., Toxicol In Vitro. 2011 Dec;25(8): 1855-62). In the context of the invention the release of lactate dehydrogenase (LDH) from a cell can be determined by any suitable method known in the art, e.g. by measuring lactate in supernatant medium using a colorimetric assay as originally described by Babson (Babson and Phillips, 1965; Clin Chim Acta 12, 210-215). Preferably, lactate dehydrogenase (LDH) activity is determined using a colorimetric assay kit for the quantification of lactate dehydrogenase (LDH) activity as provided by various suppliers, e.g. by Roche Diagnostics GmbH, Germany.

The present inventors unexpectedly found that LDH release from cells and, in particular, from skin cells can be effectively reduced by treating these cells with a gluconic acid salt. Specifically, sodium gluconate has been demonstrated to significantly reduce the concentration of LDH in the culture medium of human fibroblasts and keratinocytes *in vitro* (see Fig. 1 and Fig. 2). It was particularly unexpected that said gluconic acid salt was already effective at very low concentrations by weight. Specifically, the reduction of LDH release as marker for reduced cellular stress was already observed at a sodium gluconate concentration in the cell culture medium of about 0.05% by weight.

Accordingly, in the context of the present invention, the gluconic acid salt is preferably used at a concentration of from 0.01 to 10% by weight, from 0.05 to 9% by weight, from 0.1 to 6% by weight, from 0.5 to 4% by weight, or from 1 to 2% by weight of said composition, and preferably from 0.05 to 0.1% by weight or from 0.5 to 1% by weight of said composition.

In a further preferred embodiment a "cell" within the meaning of the present invention is a skin cell, such as a keratinocyte, fibroblast or endothelial cell, and preferably a keratinocyte or a fibroblast. It is preferred that the skin cell is a human skin cell.

### Pharmaceutical composition

The pharmaceutical composition according to the invention can be provided in any pharmaceutical dosage form known in the art. Preferably, said pharmaceutical dosage form is suitable for non-systemic, i.e. local administration to a subject's body. Such pharmaceutical dosage forms may be selected from a cutaneous cream (W/O, O/W, W/OW), a cutaneous ointment, a cutaneous gel, a cutaneous plaster, a cutaneous foam, a cutaneous spray, a cutaneous paste, a cutaneous powder, a cutaneous emulsion, a cutaneous solution, a cutaneous suspension, a solution for injection, a suspension for injection, an emulsion for injection, and a gel for injection.

In a particularly preferred embodiment, the pharmaceutical composition of the invention is provided in a pharmaceutical dosage form suitable for topical administration to a subject's body. Such dosage forms for topical administration are preferably selected from a cutaneous cream a cutaneous ointment, a cutaneous gel, a cutaneous plaster, a cutaneous foam, a cutaneous spray, a cutaneous paste, a cutaneous powder, a cutaneous emulsion, a cutaneous solution, and a cutaneous suspension.

In another particularly preferred embodiment, the pharmaceutical composition of the invention is provided in a pharmaceutical dosage form suitable for injection into the skin, preferably for injection into the epidermis, dermis or subcutis, more preferably for injection into the dermis, and most preferably for injection into the *stratum reticulare* of the subject. Such dosage forms are preferably selected from a solution for injection, a suspension for injection, an emulsion for injection, and a gel for injection. In a preferred embodiment, the dosage form suitable for injection into the skin is a gel for injection, such as a dermal filler composition.

It is also preferred that the pharmaceutical composition is a slow-release composition, which is designed to release the gluconic acid salt form the composition slowly over an extended period of time. This can be achieved through a variety of formulations and methods known in the art, for example liposomal formulations, micro-encapsulation, hydrogels, transdermal patches and matrix systems including hydrophobic matrices, lipid matrices, hydrophilic matrices, biodegradable matrices, and mineral matrices, etc.

It is preferred that the gluconic acid salt is comprised in the pharmaceutical composition of the invention at a concentration of from 0.01 to 10% by weight, from 0.05 to 9% by weight, from 0.1 to 6% by weight, from 0.5 to 4% by weight, or from 1 to 2% by weight of said composition, and preferably from 0.05 to 0.1% by weight or from 0.5 to 1% by weight of said composition.

The pharmaceutical composition according to the invention may comprise further pharmaceutically acceptable components such as hyaluronic acid, collagen, nicotin amide, provitamin B5, moringa extract, vitamin E, a UV filter, and combinations thereof. It is particularly preferred that the pharmaceutical composition comprises hyaluronic acid. In a further embodiment, the pharmaceutical composition of the invention further comprises vitamin C and/ or vitamin A, and preferably all trans retinyl palmitate. In a particular embodiment, the pharmaceutical composition of the invention comprises vitamin C, vitamin A and/or hyaluronic acid. Alternatively or in addition said composition may comprise a natural oil component selected from soy oil, argan oil, shea butter, almond oil and combinations thereof.

In order to further enhance the effects of the gluconic acid salt, the pharmaceutical composition may further comprise a pharmaceutically acceptable agent for enhancing the skin penetration of said gluconic acid salt. Suitable penetration enhancers are preferably selected from the group consisting of a C12-C15 alkyl benzoate, pentylene glycol, polyethylene glycol, ethoxydiglycol, dimethyl sulfoxide, sodium lauryl sulfate, polysorbate polyethylene sorbitane monolaurate, lecithin and mixtures thereof. Finally, the pharmaceutical composition of the invention may further comprise a preserving agent, which is preferably selected from the group consisting of phenoxyethanol, benzoic acid, dehydroacetic acid, ethylhexylglycerin, sodium benzoate and combinations thereof.

In a preferred embodiment, the pharmaceutical composition of the present invention comprises sodium gluconate, a natural oil component selected from soy oil, argan oil, shea butter, almond oil and combinations thereof, and/or a further component selected from hyaluronic acid, collagen, nicotin amide, provitamin B5, moringa extract, vitamin E, a UV filter, and combinations thereof.

Each of the herein described components may be present in the pharmaceutical composition in a concentration from 0.001% to 12%, from 0.01% to 10%, from 0.1% to 10%, from 1% to 8%, from 2% to 5% or from 3% to 4% per weight of said composition.

### Use of the pharmaceutical composition

The advantageous effects of the inventive composition in treating an age-associated condition caused by cellular stress in a subject are particularly evident when the composition is applied onto or into the skin of said subject. Therefore, in a preferred embodiment, the pharmaceutical composition of the invention is topically administered to the subject.

It is particularly preferred that said topical administration comprises applying the pharmaceutical composition of the invention to the surface of the skin. The skin area on which the pharmaceutical composition is applied is preferably selected from the face, neck, trunk or limbs of the subject's body.

It is further preferred that said topical administration comprises applying the composition of the invention on a daily basis, preferably 1x, 2x or 3x daily, and/or over a prolonged period of time, preferably for at least one week, more preferably for at least two weeks, and even more preferably for at least one month, for at least one year or for at least five years.

The inventive composition is typically used in treating an aging subject, and, preferably, an aging human. In the present context, the term "aging human" is intended to refer to a human who is at least 30 years, preferably at least 40 years, more preferably at least 50 years, even more preferably at least 60 years, in particular at least 70 years, or even at least 80 years of age.

Further, the treated subject is typically at risk for developing or afflicted with an age-associated condition. Preferably, said age-associated condition is an age-related skin condition or disease, such as dry skin, itching, ulcers, fungal infections, benign and malignant tumors, and combinations thereof.

In an alternative embodiment, a method of cosmetically treating a healthy area of skin of a subject with the pharmaceutical composition of the present invention is provided, preferably wherein said individual is an aging human subject within the meaning of the present invention.

The term " healthy area of skin" as used herein refers to a skin area which is not afflicted with any disease, disorder or pathological condition which commonly requires therapeutic treatment (e.g. infections, papulose-squamous skin diseases, urticaria, erythema, dermatitis solaris acuta, rosacea, purpura actinica, and pigmentation disorders such as melasma of a certain severity that commonly requires therapeutic treatment). Healthy skin is preferably intact, unwounded, has no haemorrhages and/or haematomas, and is dermatologically unremarkable. Healthy skin can also be called "physiologically normal skin". The loss of elasticity, firmness and/or tonicity of the skin and/or the formation of wrinkles associated with normal ageing is not to be considered as a disease, disorder or pathological condition.

The terms "cosmetically treat" or "cosmetic treatment" refer to the contacting of a subject with a cosmetic agent, preferably by topical application to the skin of the individual. A "cosmetic treatment", in contrast to a therapeutic treatment, has the effect of preventing, mitigating and/or correcting a cosmetically undesirable condition such as signs of skin ageing, and in particular of wrinkles, loss of elasticity, firmness and/or tonicity of the skin

In preferred embodiments of the above uses and methods, the pharmaceutical compositions injected into the epidermis, dermis or subcutis of the subject, preferably into the dermis, and most preferably into the *stratum reticulare.* Said injection is particularly effective in treating sunken scars, such as acne scars, and to fill smaller soft tissue defects, for example after a tumor disease. Alternatively, said injection can be used for aesthetic corrections, such as for the correction of moderate to severe facial wrinkles and skin folds, such as nasolabial folds. In both cases, the pharmaceutical composition of the invention is preferably used in form of a dermal filler composition.
The invention is described by way of the following examples which is to be construed as merely illustrative and not limitative of the scope of the invention.

### EXAMPLE 1

The efficiency of a gluconic acid salt in treating cellular stress was tested in cultured primary human fibroblast cells derived from an aging donor by quantification of lactate dehydrogenase (LDH) release from these cells. Therefore, pooled fibroblasts from three donors (one male, two female, average age 54.67, range 46-63) have been cultivated in DMEM until 80% of confluence was reached. LDH release after 24h of incubation with sodium gluconate (SG) was measured according to manufacturer's instructions (Roche, Cat. 11644793001) and compared to unexposed fibroblasts in DMEM (baseline control). Therefore, cells were incubated at 37°C under 5% CO₂ atmosphere with a SG solution in DMEM at a concentration of 1.25% and 0.625% by weight of total DMEM in the culture, respectively. As a baseline control, cells were incubated with the corresponding amount of SG free DMEM. The absorbance of the samples was measured on a standard microplate reader at 490 nm.

### Results

Significantly reduced levels of LDH were detected in fibroblast cultures after 24h treatment with SG already at the lower concentration of SG of 0.625% by weight, compared to the baseline control (see **Fig. 1**).

### Discussion

Cultured fibroblast, like fibroblasts *in vivo,* are permanently subject to cellular stress caused by aging. Since cellular stress caused by aging is associated with a loss of membrane integrity, the release of LDH increases as a function of the age of the fibroblast. The present results demonstrate that treating human fibroblasts with a solution of sodium gluconate significantly reduces LDH release from these cells, which indicates that sodium gluconate is highly effective in treating cellular stress.

### EXAMPLE 2

The efficiency of a gluconic acid salt in treating cellular stress was tested in cultured primary human keratinocyte cells derived from an aging donor by quantification of lactate dehydrogenase (LDH) release from these cells. Therefore, pooled keratinocytes from three donors (one male, two female, average age 54.67, range 46-63) have been cultivated in DMEM until 80% of confluence was reached. LDH release after 24hrs of incubation with sodium gluconate (SG) was measured according to manufacturer's instructions (Roche, Cat. 11644793001) and compared to unexposed fibroblasts in DMEM (baseline control). Therefore, cells were incubated at 37°C under 5% CO₂ atmosphere with a SG solution in DMEM at a concentration of 1.25% and 0.0781% by weight of total DMEM in the culture, respectively. As a baseline control, cells were incubated with the corresponding amount of SG free DMEM. The absorbance of the samples was measured on a standard microplate reader at 490 nm.

### Results

Significantly reduced levels of LDH were detected in keratinocyte cultures after 24hrs treatment with SG already at the lower concentration of SG of 0.0781% by weight, compared to the baseline control (see **Fig. 2**).

### Discussion

Cultured keratinocytes, just like keratinocytes *in vivo,* are permanently subject to cellular stress caused by aging. Since cellular stress caused by aging is associated with a loss of membrane integrity, the release of LDH increases as a function of the age of the keratinocytes. The present results demonstrate that treating human keratinocytes with a solution of sodium gluconate significantly reduces LDH release from these cells, which indicates that sodium gluconate is highly effective in treating cellular stress.

## Claims

1. Pharmaceutical composition comprising a gluconic acid salt for use in treating an age-associated condition caused by cellular stress in a subject.

2. Pharmaceutical composition for use according to claim 1, wherein cellular stress comprises release of lactate dehydrogenase (LDH) from a cell, preferably wherein said cell is a skin cell, more preferably a keratinocyte, fibroblast and/or endothelial cell.

3. Pharmaceutical composition for use according to claim 1 or 2, wherein said use comprises topically administering the pharmaceutical composition to the subject.

4. Pharmaceutical composition for use according to claim 1 or 2, wherein said use comprises injecting the pharmaceutical composition into the epidermis, dermis or subcutis of the subject, preferably into the dermis, most preferably into the *stratum reticulare.*

5. Pharmaceutical composition for use according to any one of the preceding claims, wherein the gluconic acid salt is sodium gluconate.

6. Pharmaceutical composition for use according to any one of the preceding claims, wherein the gluconic acid salt is comprised in the composition at a concentration of from 0.01 to 10% by weight, from 0.05 to 9% by weight, from 0.1 to 6% by weight, from 0.5 to 4% by weight, or from 1 to 2% by weight of said composition, and preferably from 0.05 to 0.1% by weight or from 0.5 to 1% by weight of said composition.

7. Pharmaceutical composition for use according to any one of the preceding claims, wherein said composition is selected from the group consisting of a cutaneous cream, a cutaneous ointment, a cutaneous gel, a cutaneous plaster, a cutaneous foam, a cutaneous spray, a cutaneous paste, a cutaneous powder, a cutaneous emulsion, a cutaneous solution, a cutaneous suspension, a solution for injection, a suspension for injection, an emulsion for injection, and a gel for injection.

8. Pharmaceutical composition for use according to any one of the preceding claims, wherein said composition is a slow-release composition.

9. Pharmaceutical composition for use according to any one of the preceding claims, wherein said composition further comprises a natural oil component selected from soy oil, argan oil, shea butter, almond oil and combinations thereof, and/or a further component selected from hyaluronic acid, collagen, nicotin amide, provitamin B5, moringa extract, vitamin E, a UV filter, and combinations thereof.

10. Pharmaceutical composition for use according to any one of the preceding claims, wherein said composition further comprises vitamin C and/ or vitamin A, preferably all trans retinyl palmitate.

11. Pharmaceutical composition for use according to any one of the preceding claims, wherein said composition further comprises a preserving agent, preferably selected from the group consisting of phenoxyethanol, benzoic acid, dehydroacetic acid, ethylhexylglycerin, sodium benzoate and combinations thereof.

12. Pharmaceutical composition for use according to any one of the preceding claims, wherein said composition further comprises a skin penetration enhancing agent, preferably selected from the group consisting of a C₁₂-C₁₅ alkyl benzoate, pentylene glycol, polyethylene glycol, ethoxydiglycol, dimethyl sulfoxide, sodium lauryl sulfate, polysorbate polyethylene sorbitane monolaurate, lecithin and mixtures thereof.

13. Pharmaceutical composition comprising sodium gluconate, a natural oil component selected from soy oil, argan oil, shea butter, almond oil and combinations thereof, and/or a further component selected from hyaluronic acid, collagen, nicotin amide, provitamin B5, moringa extract, vitamin E, a UV filter, and combinations thereof.

14. Pharmaceutical composition according to claim 13, wherein sodium gluconate is comprised in the composition at a concentration of from 0.01 to 10% by weight, from 0.05 to 9% by weight, from 0.1 to 6% by weight, from 0.5 to 4% by weight, or from 1 to 2% by weight of said composition, and preferably from 0.05 to 0.1% by weight or from 0.5 to 1% by weight of said composition.

15. Method of cosmetically treating a healthy area of skin of a subject with a pharmaceutical composition according to claim 13 or 14, preferably wherein said subject is an aging subject.
